Europäisches Patentamt

European Patent Office

Office européen des brevets

(19)

(11) **EP 1 332 721 A1**

(12) # EUROPEAN PATENT APPLICATION

(43) Date of publication:
06.08.2003 Bulletin 2003/32

(51) Int Cl.7: **A61B 6/00**

(21) Application number: 02004025.9

(22) Date of filing: 22.02.2002

(84) Designated Contracting States:
**AT BE CH CY DE DK ES FI FR GB GR IE IT LI LU MC NL PT SE TR**
Designated Extension States:
**AL LT LV MK RO SI**

(30) Priority: 04.02.2002 EP 02002556

(71) Applicant: **Rust, Georg-Friedemann, Dr.**
**82131 Gauting (DE)**

(72) Inventor: **Rust, Georg-Friedemann, Dr.**
**82131 Gauting (DE)**

(74) Representative: **Grünecker, Kinkeldey,**
**Stockmair & Schwanhäusser Anwaltssozietät**
**Maximilianstrasse 58**
**80538 München (DE)**

(54) **Method for reducing dose and/or exposure time in medical imaging**

(57) The invention is directed to a method for reducing dose and/or exposure time in medical imaging comprising the steps of determining a reduced dose and/or exposure time in accordance with a predetermined signal-to-noise ratio, the dose and/or exposure time being reduced with respect to standard conditions, ofcreating image data with said reduced dose and/or exposure time, and of improving the signal-to-noise ratio of the image data.

Fig. 1

**Description**

**Description of the Invention**

The invention is directed to a method for reducing dose and/or exposure time in medical imaging.

In medicine, several methods are known to image inner parts of the human body that are not visible from outside the body. These methods are based on different physical phenomena: Electro-magnetic radiation is used in the case of X-ray imaging, in particular, of Computed Tomography (CT) but also in resonance methods as, for example, Magnetic Resonance Imaging (MRI), Nuclear Magnetic Resonance (NMR) or Magnetic Resonance Tomography (MRT). Positron Emission Tomography (PET) and Radionuclide Imaging, e.g., are based on radioactive decay whereas Sonography uses ultrasound, to mention only some examples of medical imaging. All these methods have in common that the target, in the medical case the human body or parts of it, is exposed for a certain exposure time. During the exposure, energy, e.g. in form of radiation or acoustic waves, is transferred to the target and partly absorbed. The absorbed energy, which might be normalized per volume, mass and/or time, is called "dose". In the case of X-rays, for example, one considers the amount of ionizing radiation that is absorbed in the target per unit mass. The biological effect of the absorbed energy is characterized by the equivalent dose that is given by the product of a weighting factor and the energy dose. In image based diagnostics, it is the first aim to obtain images with high resolution and contrast in order to clearly detect anomalies and pathologies. In particular, a high signal-to-noise ratio (SNR) is desirable. Depending on the imaging method and on the target object, for example, the organ to be examined, standard parameters (standard conditions) have been established to obtain standard image quality. It is to be noted that CT examinations, for example, of targets with high contrast (coloscopy, examination of thorax or bone, e.g.) require completely different parameters than examinations of targets with low contrast (abdomen, heart or mediastinum, e.g.). CT examinations of high contrast targets (bone, transition air/soft tissue, e.g.) are usually performed using tube currents of about 100 mAs, whereas low contrast soft tissue targets are usually radiated using tube currents of about 200 mAs.

The prior art techniques suffer from several drawbacks. First of all, to achieve the goal of high resolution and contrast, the imaging is performed using high intensity and/or long exposure time. In particular, when using digital solid state detectors for X-ray detection or when using SQUIDs for the detection of magnetic fields, higher dose and/or exposure time is required to obtain similar image quality than for conventional films or detectors. Furthermore, an increase of the spatial resolution, e.g. by increasing the number (per area or volume) of voxels or pixels, necessitates an increase of intensity. The resulting dose and/or exposure time in all or almost all methods results in damages of the target and its surroundings. The damaging effects of X-rays and radioactive radiation are well known. But also the influence of strong magnetic fields for a long time and the resulting warming of the patient in the case of resonance methods does not seem to be completely harmless. This is also the case for ultrasound waves, in particular, when applied to an embryo or fetus in the womb.

In addition, a high intensity increases the wastage of the used appartus, both source and detector, and results in a shorter lifetime thereof.

It is therefore the problem underlying the invention to overcome these drawbacks and to provide a method and apparatus for reducing dose and/or exposure time in medical imaging while maintaining or even improving the image quality.

The problem is solved by the method of claim 1. Accordingly, a method is provided for reducing dose and/or exposure time in medical imaging comprising the steps:

a) determining a reduced dose and/or exposure time in accordance with a predetermined signal-to-noise ratio, the dose and/or exposure time being reduced with respect to standard conditions,

b) creating image data with said reduced dose and/or exposure time, and

c) improving the signal-to-noise ratio of the image data.

Due to the reduction of dose and/or exposure time, damages of the target can be reduced or even eliminated. In particular, in step a) the dose and/or exposure time can be reduced until an expected signal-to-noise ratio is very low but still sufficient to again improve the image data in step c) such that a necessary resolution and/or contrast is obtained.

On the other hand, the method allows for an increase of spatial or temporal image resolution without increase of dose and/or exposure time. This is equivalent to reducing the dose and/or exposure time with respect to the standard conditions of the higher resolution. This is particularly useful when using a digital solid state detector for detecting X-rays.

An additional advantage is the reduced use of the imaging apparatus per image which can result in an improved lifetime. In magnetic resonance methods, in particular, when decreasing the exposure time, the rate of target objects to be examined can be increased. If the resulting image data is stored in digital form, the improving of the signal-to-noise ratio allows for a more efficient compression of the data which is useful, for example, in teleradiology.

The step of improving the signal-to-noise ratio can be performed on the basis of the raw image data or on the basis of processed image data.

It is to be understood that the method of the invention is not restricted to medical imaging but can also be used for any other target.

Preferably, the medical imaging comprises radiating with X-rays, applying radionuclides, applying magnetic fields, applying ultrasound and/or injecting a contrast agent. Possible contrast agents are, for example, iodine containing substances (for CT or angiography), paramagnetic substances such as a Gadolinium-compound (for Magnetic Resonance) or gas-filled microbubbles (for ultrasound).

Advantageously, the reduced dose and/or exposure time is determined using a predetermined relation between dose and/or exposure time and signal-to-noise ratio. Using such a predetermined relation has the advantage that it is not necessary to perform corresponding tests with each target. Such a relation can be determined theoretically or by performing a series of tests, for example.

In a preferred embodiment of all methods described above, in step b) low contrast image data or high contrast image data are created. It is to be noted that these two alternatives are very different due to the different kinds of tissue that are examined.

Step c) can comprise the step of filtering the image data, preferably using a nonlinear filter, for example, a weighted median filter. In this way, the image data can be smoothed, thus, improving the signal-to-noise ratio. In particular, a nonlinear filter is preferred when improving processed image data; especially in the case of improving raw image data, a suitable alternative is given by linear filters. In particular, step c) can also comprise a combination of different filtering methods. In addition, it is advantageous to further optimize the parameters of the employed filters during the filtering of the data.

To avoid the smoothing of edges, an edge preserving smoothing algorithm is preferred. Using such a smoothing algorithm, edges (e.g. of organs or other structures) can be preserved and even accentuated with negligible image deterioration. Advantageously, the edge preserving smoothing algorithm comprises a nonlinear Gaussian filter chain. Alternative methods comprise, for example, anisotropic diffusion, wavelets or gradient methods.

It is particularly useful to employ filters that are not restricted to a special dimension. Gaussian filters, for example, can be used in arbitrary dimension. With such filters, the method of the invention can be used to obtain image sequences with improved spatial resolution and improved time resolution without increasing the dose and/or exposure time. In this case of functional imaging, the filtering is to be performed in a four-dimensional space.

The invention also provides an apparatus for performing steps a) and c) of any of the above described methods comprising means for determining reduced dose and/or exposure time in accordance with a predetermined signal-to-noise ratio and means for reducing the signal-to-noise ratio of the image data. Preferably, a system is provided comprising the aforementioned apparatus and imaging means for creating image data with reduced dose and/or exposure time. The apparatus can be a computer, in particular a parallel computer, which is connected to an imaging means, for example, a (spiral) CT-apparatus. The same computer can be used to drive the imaging means.

In addition, the invention provides a computer program product directly loadable into the internal memory of a digital computer, comprising software code portions for performing steps a) and c) of the above-described method, when said product is run on a computer.

The invention also provides a computer program product stored on a medium readable by a computer, comprising computer readable program means for causing a computer to perform steps a) and c) of the method described above, when said product is run on a computer.

Preferably, the aforementioned computer program product further comprises software code portions for driving imaging means for creating image data with reduced dose and/or exposure time and for processing said image data to be used for improving the signal-to-noise ratio of the image data, when said product is run on a computer.

To reduce the required time for improving the image data, it is advantageous to use several computer processors in parallel and a corresponding parallelized computer program.

Further features and advantages of the invention are described in the context of the following examples and with reference to the drawings wherein

Fig. 1    shows the results of computer simulations for image data with different signal-to-noise ratio and the corresponding improved image data and

Fig. 2    shows a model for a human colon using a pork intestine and corresponding CT images for normal dose and low dose.

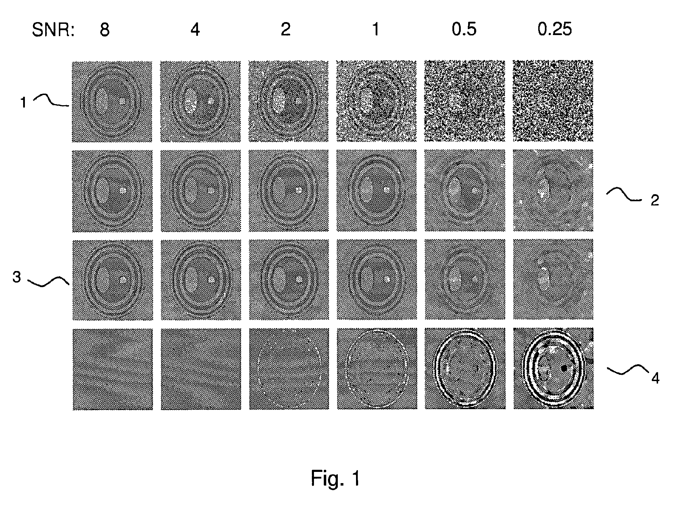

The efficiency of the method of the invention is illustrated in Fig. 1 using computer simulations based on 3D geometry primitives. Four ellipsoidal shells with different thicknesses were places in a cube of $128^3$ voxels. The thickness of the shells varied from 1 to 5 pixels from the outside to the inside. To simulate reduced dose and/or exposure time, the datasets were randomized by adding white Gaussian noise (SNR between 8 and 0.25 with SNR=D/σ ≡ Contrast/std. dev.) yielding the images in the upper line 1. The second line 2 shows the resulting improved image after four iterations of the nonlinear gaussian filter chain and the third line 3 the result of five iterations. The lowest line 4 presents the differential images obtained by subtracting the filtered image from the noisy data. An image can be modelled as a grey value function $f(q): P \rightarrow \mathbb{R}_+$ with $P \subset \mathbb{R}^3$ the set of image points. A filtered image using a nonlinear Gaussian filter chain is then given by:

$$G_{\sigma_x,\sigma_z} f(p) = \frac{1}{N_p} \sum_{q \in P} g_{\sigma_x} (\| q - p \|) \, g_{\sigma_z} (f(q) - f(p)) \, f(q), \qquad .$$

with the Gaussian weight

$$g_\sigma(t) = \exp\left( -\frac{t^2}{2\sigma^2} \right)$$

and

$$N_p = \sum_{q \in P} g_{\sigma_x} (\| q - p \|) \, g_{\sigma_z} (f(q) - f(p)).$$

As can be seen in Fig. 1, the filtering reconstructs the image even for an SNR between 1 and 0.5, for which in the noisy image the structure is barely recognizable. For this SNR range, the loss of image morphology is restricted to structures of 1 to 3 pixels in size, which corresponds to a real size of 0.6 mm (SNR = 1) to 1.8 mm (SNR = 0.5) in the x - y direction if a 512 x 512 matrix is used. Thus, no clinically relevant image information is lost. Therefore, by using the approximation for CT imaging

$$\text{SNR} \sim \sqrt{\text{SL} \cdot \text{I}}$$

with SL the slice thickness and I the tube current, one can determine a corresponding reduced dose for which an image, being improved in accordance with the method of the invention, still displays all clinically relevant information.

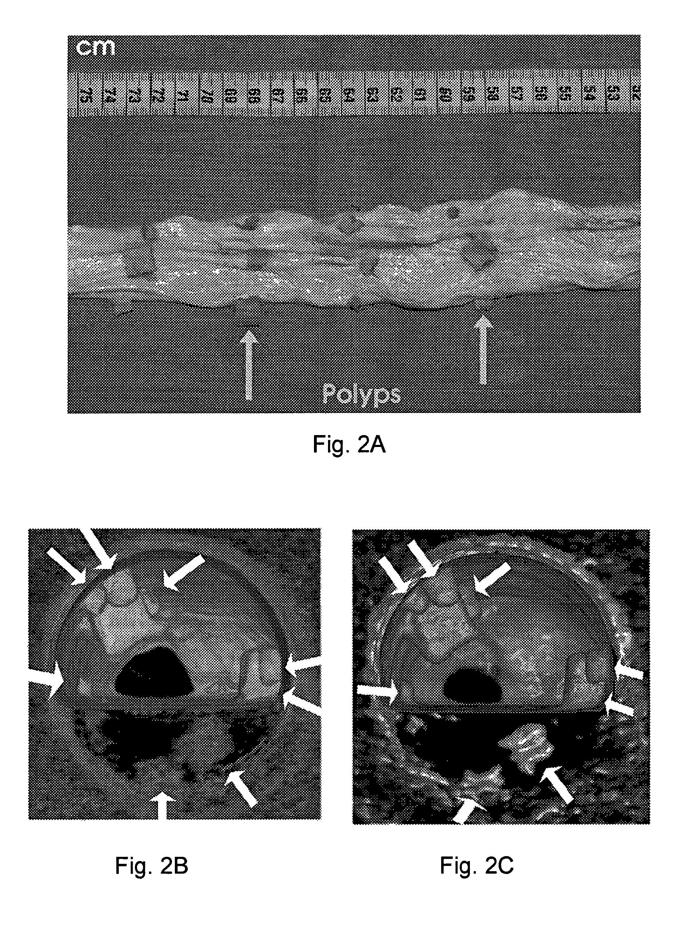

Fig. 2A shows an arrangement of a pork intestine with meat particles sewed on the mucosa of the colon wall. The meat particles have edge lengths of 3 mm, 6 mm, 9 mm, and 12 mm. The colon model was placed in a water bath to simulate the same absorbtion conditions as found in patients of normal weight (75 kg). The intestine was filled with ambient air and then with liquid up to a third of its height. CT images were taken using the following acquisition and reconstruction parameters: voltage 120 keV, collimation 4 x 1 mm, revolution time 0.5 s, feed 6 mm/revolv., slice thickness 1.25 mm, slice overlap 50 % and a soft tissue reconstruction kernel. Fig. 2B shows a virtual endoscopic view taken at a standard tube current of 140 mAs. Other image data were obtained using an extremely low dose current of 20 mAs. The image data have been improved using a nonlinear Gaussian filter chain resulting in the virtual endoscopic view shown in Fig. 2C.

**Claims**

1.   A method for reducing dose and/or exposure time in medical imaging comprising the steps:

   a) determining a reduced dose and/or exposure time in accordance with a predetermined signal-to-noise ratio, the dose and/or exposure time being reduced with respect to standard conditions,

b) creating image data with said reduced dose and/or exposure time, and

c) improving the signal-to-noise ratio of the image data.

2.  The method of claim 1, wherein the medical imaging comprises radiating with X-rays, applying radionuclides, applying magnetic fields, applying ultrasound and/or injecting a contrast agent.

3.  The method of one of the preceding claims, wherein the reduced dose and/or exposure time is determined using a predetermined relation between dose and/or exposure time and signal-to-noise ratio.

4.  The method of one of the preceding claims, wherein in step b) low contrast image data or high contrast image data are created.

5.  The method of one of the preceding claims, wherein step c) comprises the step of filtering the image data, preferably using a nonlinear filter.

6.  The method of claim 5, wherein the filtering is performed using an edge preserving smoothing algorithm, preferably using a nonlinear Gaussian filter chain.

7.  An apparatus for perfoming steps a) and c) of the method of one of the preceding claims comprising means for determining reduced dose and/or exposure time in accordance with a predetermined signal-to-noise ratio and means for reducing the signal-to-noise ratio of the image data.

8.  A system for performing the method of one of the claims 1 - 6 comprising the apparatus of claim 7 and imaging means for creating image data with reduced dose and/or exposure time.

9.  A computer program product directly loadable into the internal memory of a digital computer, comprising software code portions for performing steps a) and c) of the method of claims 1- 7, when said product is run on a computer.

10. A computer program product stored on a medium readable by a computer, comprising computer readable program means for causing a computer to perform steps a) and c) of the method of claims 1 - 7, when said product is run on a computer.

11. The computer program product of claims 9 or 10, further comprising software code portions for driving imaging means for creating image data with reduced dose and/or exposure time and for processing said image data to be used for improving the signal-to-noise ratio of the image data, when said product is run on a computer.

Fig. 1

Fig. 2A

Fig. 2B

Fig. 2C

European Patent
Office

**PARTIAL EUROPEAN SEARCH REPORT**

which under Rule 45 of the European Patent Convention
shall be considered, for the purposes of subsequent
proceedings, as the European search report

Application Number

EP 02 00 4025

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | RAM G: "Optimization of ionizing radiation usage in medical imaging by means of image enhancement techniques" MEDICAL PHYSICS, SEPT.-OCT. 1982, USA, vol. 9, no. 5, pages 733-737, XP002241834 ISSN: 0094-2405 | 1,2,4,5, 7-11 | A61B6/00 |
| A | * page 733 - 734, section "Image Quality" * <br> * page 735, left-hand column, line 23 - 28 * <br><br> --- | 3,6 | |
| Y | MOTZ J W ET AL: "Image information content and patient exposure (in radiography)" MEDICAL PHYSICS, JAN.-FEB. 1978, USA, vol. 5, no. 1, pages 8-22, XP001149936 ISSN: 0094-2405 * page 15, right-hand column, line 4 from bottom - page 16, left-hand column, line 14 * <br> * page 18, left-hand column, line 6 - 15 * <br> --- <br> -/-- | 1-5,7-11 | |

TECHNICAL FIELDS
SEARCHED (Int.Cl.7)

A61B
G06T

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 30 May 2003 | Knüpling, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent
Office**

INCOMPLETE SEARCH
SHEET C

Application Number

EP 02 00 4025

In claim 2, the step of applying radionuclides or injecting a contrast agent is considered as a method of treatment of the human body by surgery which is exempted from patentability according to Article 52(4) EPC. Consequently, the search was restricted to the claim as follows: The method of claim 1, wherein the medical imaging comprises radiating with X-rays, applying magnetic fields, and/or applying ultrasound.

-------------------

Claim(s) searched incompletely:
        2

Reason for the limitation of the search (non-patentable invention(s)):

Article 52 (4) EPC - Method for treatment of the human or animal body by surgery

European Patent
Office

**PARTIAL EUROPEAN SEARCH REPORT**

Application Number

EP 02 00 4025

| | **DOCUMENTS CONSIDERED TO BE RELEVANT** | | CLASSIFICATION OF THE APPLICATION (Int.Cl.7) |
|---|---|---|---|
| Category | Citation of document with indication, where appropriate, of relevant passages | Relevant to claim | |
| Y | LA RIVIERE P J ET AL: "Improved detectability of malignant lesions in SPECT scintimammography using effective multi-dimensional smoothing" NUCLEAR SCIENCE SYMPOSIUM, 1997. IEEE ALBUQUERQUE, NM, USA 9-15 NOV. 1997, NEW YORK, NY, USA,IEEE, US, 9 November 1997 (1997-11-09), pages 1581-1585, XP010275671 ISBN: 0-7803-4258-5 * page 1585, left-hand column, line 2 - 17 * | 1-5,7-11 | |
| | --- | | |
| A | KITAMURA K ET AL: "Noise reduction in PET attenuation correction using non-linear Gaussian filters" NUCLEAR SCIENCE SYMPOSIUM, 1999. CONFERENCE RECORD. 1999 IEEE 24-30 OCTOBER 1999, PISCATAWAY, NJ, USA,IEEE, US, 24 October 1999 (1999-10-24), pages 962-966, XP010500631 ISBN: 0-7803-5696-9 * page 962, left-hand column, section "Introduction", line 5 - 6 * * page 962, right-hand column, line 18 - 20 * | 1,3-11 | TECHNICAL FIELDS SEARCHED (Int.Cl.7) |
| | --- | | |
| | -/-- | | |

EPO FORM 1503 03.82 (P04C10)

**European Patent Office**

## PARTIAL EUROPEAN SEARCH REPORT

Application Number

EP 02 00 4025

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | CHAN C L ET AL: "IMAGE SEQUENCE FILTERING IN QUANTUM-LIMITED NOISE WITH APPLICATIONSTO LOW-DOSE FLUOROSCOPY" IEEE TRANSACTIONS ON MEDICAL IMAGING, IEEE INC. NEW YORK, US, vol. 12, no. 3, 1 September 1993 (1993-09-01), pages 610-621, XP000412339 ISSN: 0278-0062 * page 611, right-hand column, line 7 - 20 * * page 618 - 619, section V "Experimental Results" --- | 1,2,4,5, 7-11 | |
| A | US 4 907 156 A (DOI KUNIO ET AL) 6 March 1990 (1990-03-06) * column 13, line 57 - column 14, line 3 * --- | 1-11 | **TECHNICAL FIELDS SEARCHED** (Int.Cl.7) |
| A | SANDBORG M ET AL: "Demonstration of correlations between physical and clinical image quality measures in chest and lumbar spine radiography" PROCEEDINGS OF THE 22ND ANNUAL EMBS INTERNATIONAL CONFERENCE, CHICAGO, USA, vol. 4, 23 - 28 July 2000, pages 3078-3081, XP010531293 * page 3078, section "Introduction" * * page 3080, section "Results and Discussion", line 1 -11 * ----- | 1,7-11 | |

EPO FORM 1503 03.82 (P04C10)

# EP 1 332 721 A1

### ANNEX TO THE EUROPEAN SEARCH REPORT
### ON EUROPEAN PATENT APPLICATION NO.

EP 02 00 4025

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2003

| Patent document cited in search report | Publication date | Patent family member(s) | Publication date |
|---|---|---|---|
| US 4907156 A | 06-03-1990 | NONE | |

EPO FORM P0459

For more details about this annex : see Official Journal of the European Patent Office, No. 12/82

12